# EUROPÄISCHE PATENTSCHRIFT

(11) **EP 2 001 381 B1**
(45) Veröffentlichungstag und Bekanntmachung des Hinweises auf die Patenterteilung: **16.01.2013**
(21) Anmeldenummer: 06724043.2
(22) Anmeldetag: 05.04.2006
(51) Int. Cl.: A61B 17/80, A61F 2/14, A61F 2/28, B21D 22/02, B21D 35/00

(54) **VERFAHREN UND VORRICHTUNG ZUR HERSTELLUNG EINES ENTSPRECHEND EINER ANATOMISCHEN SOLLFORM VORGEFORMTEN FLÄCHIGEN IMPLANTATS FÜR EINEN MENSCHLICHEN ODER TIERISCHEN KÖRPER**
METHOD AND DEVICE FOR PRODUCING A PLANAR IMPLANT FOR A HUMAN OR ANIMAL BODY, WHICH PLANAR IMPLANT IS PREFORMED CORRESPONDING TO A DESIRED ANATOMICAL SHAPE
PROCEDE ET DISPOSITIF DE FABRICATION D'UN IMPLANT PLAT PREFORME EN CORRESPONDANCE A UNE FORME ANATOMIQUE DE CONSIGNE POUR LE CORPS HUMAIN OU LE CORPS D'UN ANIMAL

(43) Veröffentlichungstag der Anmeldung: 17.12.2008
(73) Patentinhaber: Synthes GmbH, 4436 Oberdorf (CH)
(72) Erfinder: METZGER, Marc, Christian, 79199 Kirchzarten (DE)
(74) Vertreter: Lusuardi, Werther
(86) Internationale Anmeldenummer: PCT/EP2006/003095
(87) Internationale Veröffentlichungsnummer: WO 2007/112766

(56) Entgegenhaltungen:
- WO-A-02/092882
- WO-A-03/007831
- DE-A1- 3 425 002
- US-A- 5 766 176
- US-A- 6 008 430

## Beschreibung

### STAND DER TECHNIK

Die vorliegende Erfindung betrifft ein Verfahren und eine Vorrichtung zur Herstellung eines entsprechend einer anatomischen Sollform vorgeformten flächigen Implantats für einen menschlichen oder tierischen Körper, bei welchen ein Rohling des flächigen Implantats bereitgestellt und danach gebogen wird.

Ein solches Verfahren ist z.B. aus der US-A-6 008 430 bekannt.

Obwohl prinzipiell auf beliebige vorgeformte flächige Implantate für einen menschlichen oder tierischen Körper anwendbar, werden die in der vorliegenden Erfindung und die ihr zugrundeliegende Problematik in Bezug auf vorgeformte Osteosynthesen für die Orbita erläutert.

Zur operativen Versorgung von Frakturen in der Orbita werden unter anderem Gitter (engl.: mesh) oder Platten aus Titan oder anderen Materialien verwendet. Diese liegen in unterschiedlichen Mustern und Stärken vor, und sind in der Vertriebsform üblicherweise eben gestaltet.

Intraoperativ muss deshalb der Operateur diese Gitter von Hand zurechtschneiden, falzen und biegen, um die anatomischen Sollstrukturen nachzubilden. Hierbei ist es nicht immer möglich, die tieferen anatomischen Strukturen des Orbitatrichters zu erreichen, klar zu definieren und zu rekonstruieren. Des Weiteren ist das Ergebnis stark abhängig von der Erfahrung des Chirurgen.

Wird die Überbrückung des Defektes im tieferen Teil der Orbita nicht erreicht sind die Folgen Doppelbilder (Diplopie), Einsinken des Auges (Enopthalmus) und Motilitätstörungen. Zu große Manipulationen am Auge bzw. am Sehnerv während der Operation, können im schlimmsten Fall zu Erblindung (Amaurosis) führen, so dass zu häufiges Einpassen der Gitter in der Orbita nicht durchgeführt werden sollte.

Die der vorliegenden Erfindung zugrundeliegende Aufgabe besteht also allgemein darin, ein Verfahren und eine Vorrichtung zur Herstellung eines entsprechend einer anatomischen Sollform vorgeformten flächigen Implantats für einen menschlichen oder tierischen Körper zu schaffen, welche eine verbesserte Strukturnachbildung und eine Vereinfachung für den Operateur bilden.

### VORTEILE DER ERFINDUNG

Diese Aufgabe wird durch das erfindungsgemässe Verfahren zur Herstellung eines entsprechend einer anatomischen Sollform vorgeformten flächigen Implantats für einen menschlichen oder tierischen Körper mit den Merkmalen des Anspruchs 1, bzw. durch die entsprechende Vorrichtung nach Anspruch 12, bzw. durch die entsprechenden Verfahren nach Anspruch 20 bzw. 24 gelost. Sie weisen gegenüber den bekannten Lösungsansätzen den Vorteil auf, dass sie ein anatomisch präformiertes flächiges Implantat ermöglichen, das die anatomische Struktur ideal ersetzt oder rekonstruiert.

Mit der vorliegenden Erfindung wird erreicht, dass die operative Rekonstruktion von Körpergewebe anatomisch individueller, genauer, für den Patienten ungefährlicher, zeitlich effizienter, standardisierter und unabhängig von der Erfahrung des Chirurgen von statten geht.

Die der vorliegenden Erfindung zugrundeliegende Idee besteht darin, dass eine Vorformeinrichtung bereitgestellt wird, welche eine Mehrzahl diskreter einstellbarer Stützeinrichtungen zum Einstellen einer zugehörigen Mehrzahl von Stützpunkten entsprechend der anatomischen Sollform aufweist und an der das Bilden des vorgeformten Implantats durch Anbiegen des Rohlings an die eingestellten Stützeinrichtungen erfolgt.

Beispielsweise anhand von CT-Datensätzen werden individuelle oder geclusterte anatomische Strukturen reproduziert, die der Herstellung des präformierten Implantats über die entsprechend eingestellte Stützpunktfläche der Vorformeinrichtung dienen.

In den Unteransprüchen finden sich vorteilhafte Weiterbildungen und Verbesserungen des jeweiligen Gegenstandes der Erfindung.

Gemäss einer bevorzugten Weiterbildung weist der Rohling eine zweidimensionale Gitterstruktur auf, welche durch das Anbiegen in eine dreidimensionale Gitterstruktur überführt wird.

Gemäss einer weiteren bevorzugten Weiterbildung erfolgt das Ermitteln der Einstellungen für die Stützeinrichtungen durch folgende Schritte:
Abbilden eines der anatomischen Sollform entsprechenden Bereichs des Körpers in ein Stützpunktgitter;
Ermitteln der Abstände zwischen einer Referenzfläche entsprechend einer vorgegebenen Referenzeinstellung der Stützpunkte der Vorformeinrichtung und dem Stützpunktgitter an den Stützpunkten in der Referenzfläche bei einer vorgegebenen Orientierung des Stützpunktgitters; und
Einstellen der ermittelten Abstände an den Stützeinrichtungen.

Gemäss einer weiteren bevorzugten Weiterbildung erfolgt das Abbilden des der anatomischen Sollform entsprechenden Bereichs des Körpers in ein Stützpunktgitter durch folgende Schritte:
Abbilden eines der anatomischen Sollform gemäss einer mathematischen Abbildung entsprechenden Bereichs des Körpers in ein Abbild-Stützpunktgitter; und
Transformieren des Abbild-Stützpunktgitters in das Stützpunktgitter entsprechend der mathematischen Abbildung.

Gemäss einer weiteren bevorzugten Weiterbildung wird das Abbilden mittels einer Computertomograhie-Vorrichtung durchgeführt.

Gemäss einer weiteren bevorzugten Weiterbildung ist das vorgeformte Implantat eine vorgeformte Osteosynthese für die Orbita.

Gemäss einer weiteren bevorzugten Weiterbildung weisen die Stützeinrichtungen zylindrische Stifteinrichtungen auf, welche höhenverstellbar an einer ebenen Platteneinrichtung angebracht sind.

Gemäss einer weiteren bevorzugten Weiterbildung erfolgt das Einstellen der Stützeinrichtungen durch eine automatische Einstellungseinrichtung.

Gemäss einer weiteren bevorzugten Weiterbildung wird vor dem Anbiegen eine dünne verformbare Folieneinrichtung oder eine angeformte Manteleinrichtung auf die eingestellten Stützeinrichtungen der Vorformeinrichtung aufgebracht.

Gemäss einer weiteren bevorzugten Weiterbildung erfolgt das Vorgeben der anatomischen Sollform und das Ermitteln der entsprechenden Einstellungen für die Stützeinrichtungen durch folgende Schritte:
Vorgeben von Standard-Sollformen und entsprechenden Einstellungs-Datensätzen in einer Datenbank;
Auswählen einer Standard-Sollform aus der Datenbank anhand eines oder mehrerer folgender Parameter: Geschlecht, Alter, Rasse, Grösse, Körperseite; und
Ermitteln der entsprechenden Einstellungen für die Stützeinrichtungen durch den zu ihr ausgewählten Standard-Sollformen zugehörigen Einstellungs-Datensatz.

Gemäss einer weiteren bevorzugten Weiterbildung besteht das Implantat aus Titan. Andere Materialien bzw. die Kombination von Titan mit verschiedenen Überzügen und Beschichtungen ist möglich. Auch die Auskleidung des Gitters mit anderen Materialkompositionen, wie Kunststoff oder anderen Bioresorbierbaren Materialien, ist denkbar.

### ZEICHNUNGEN

Ausführungsbeispiele der Erfindung sind in den Zeichnungen dargestellt und in der nachfolgenden Beschreibung näher erläutert.

Es zeigen:
- Fig. 1a-d: computer-tomographisch aufgenommene Schichten einer Orbita zur Erläuterung einer Ausführungsform des erfindungsgemässen Verfahrens;
- Fig. 2a-c: zeigen verschiedene Darstellungen des bei der Ausführungsform des erfindungsgemässen Verfahrens ermittelten Stützpunktgitters für die anatomische Sollform des Implantats;
- Fig. 3: eine eingestellte Vorformeinrichtung bei der Ausführungsform des erfindungsgemässen Verfahrens;

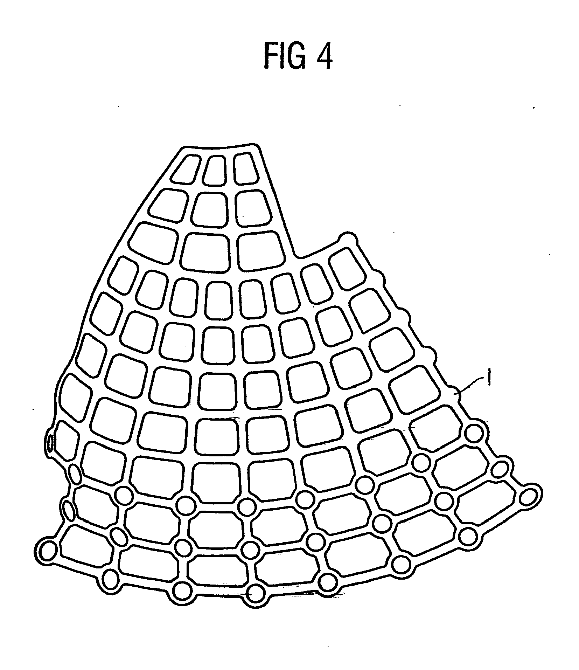

- Fig. 4: ein vorgeformtes Implantat bei der Ausführungsform des erfindungsgemässen Verfahrens; und
- Fig. 5: eine eingestellte Vorformeinrichtung bei einer weiteren Ausführungsform des erfindungsgemässen Verfahrens.

### BESCHREIBUNG DER AUSFÜHRUNGSBEISPIELE

In den Figuren bezeichnen gleiche Bezugszeichen gleiche oder funktionsgleiche Elemente.

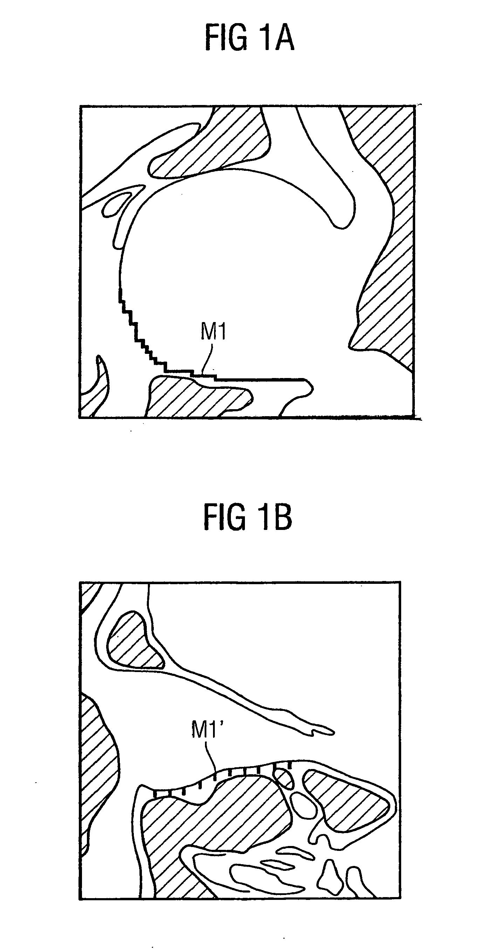

Fig. 1a-d zeigen computer-tomographisch aufgenommene Schichten einer Orbita zur Erläuterung einer Ausführungsform des erfindungsgemässen Verfahrens.

In einem ersten Schritt diesen Ausführungsform geht es darum, eine anatomische Sollform für ein vorzuformendes flächiges Implantat in Form einer Osteosynthese für die Orbita festzulegen.

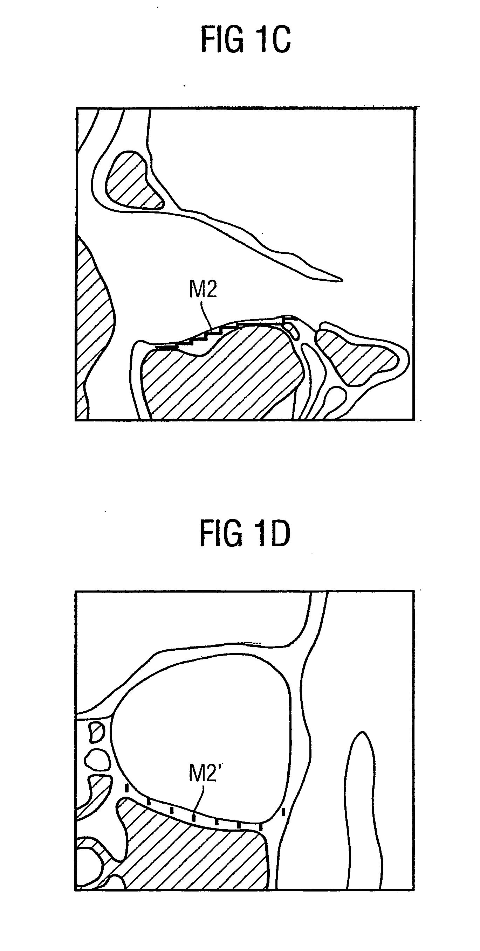

Hierzu werden, wie in Fig. 1 a-d dargestellt, computertomographische Aufnahmen verschiedener Ebenen der Orbita in verschiedenen Ansichten aufgenommen, um daraus, wie später erläutert wird, ein Stützpunktgitter zu bilden, mit dessen Hilfe sich die Einstellungen für eine Vorformeinrichtung zum Vorformen des Implantats ermitteln lassen.

Bei der vorliegenden Ausführungsform werden dabei DICOM-Daten genutzt, welche mithilfe einer entsprechenden Software dreidimensional visualisiert werden. Beispielsweise erlaubt die Software dabei folgende Ansichten:
- Dreidimensionale Ansicht des gesamten Schädels
- Koronale Ansicht (Schnitte von vorne)
- Sagittale Ansicht (Schnitte von der Seite)
- Axiale Ansicht (Schnitte in der Achse)

Zur Erfassung der Orbitabodenstruktur wird bei vorliegendem Beispiel die gesunde Schädelseite computer-tomographisch vermessen und anschliessend findet eine Spiegelung an der Schädelmitte statt. Zur späteren Bildung des Stützpunktgitters werden genau definierte Punkte benötigt, die ein Raster mit genau definiertem Abstand der Messpunkte haben. Hierzu, bedient man sich der koronalen und sagittalen Ansichten, um in definiertem Abstand Schichten zu markieren. Die Markierung kann mittels den Houndsfield-Einheiten, die die verschiedenen Grauwerte des Computertomogramms wiedergeben, automatisch durch Grauwertdefinition oder von Hand mittels eines Pinseltools erfolgen.

Bei der Markierung von Schichten in der koronalen Ansicht bietet sich als Startschicht der Bereich des vorderen Orbitarandes an. Beispielsweise hat jede Schicht eine Dicke von 0,5 mm. Durch Auswahl bzw. Überspringen bestimmter Schichten kann hierdurch eine Auflösung vorgegeben werden. So ist im Beispielsfall die höchste Auflösung ein Schichtabstand von 0,5 mm, und die geringste Auflösung lässt sich durch ein vielfaches des Schichtabstandes von 0,5 mm individuell definieren.

Bei der Markierung der Schichten in der sagittalen Ansicht, wobei vorzugsweise der gleiche Schichtabstand wie bei der koronalen Ansicht verwendet wird, bietet sich als Startschicht der Bereich des seitlichen Orbitarandes an.

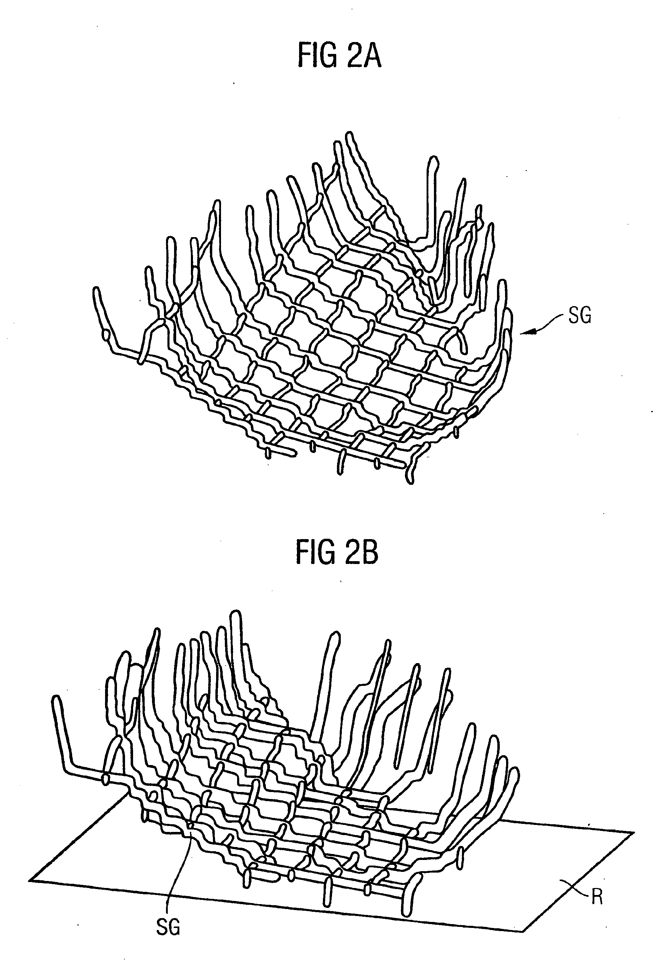

Die Markierungen M1, M2 in Abbildung 1a bzw. 1c zeigen die koronalen als auch die sagittale Markierung des Orbitabodens in seinem Verlauf in den ausgewählten Schichten. In den Abbildungen 1b und 1 d sind die entsprechenden mit Bezugszeichen M1' bzw. M2' markierten Schichten im um genau 90° gedrehten Blickwinkel dargestellt. Das Zusammensetzen der Markierungen von Abbildung 1a und 1b ergibt ein dreidimensionales Stützpunktgitter, wie nachstehend im Zusammenhang mit Fig. 2a-c erläutert.

Fig. 2a-c zeigen verschiedene Darstellungen des bei der Ausführungsform des erfindungsgemässen Verfahrens ermittelten Stützpunktgitters für die anatomische Sollform des Implantats.

Wie in Fig. 2a dargestellt, ergibt sich nach der erwähnten Spiegelung ein Stützpunktgitter SG durch die Ansicht der koronalen und sagittalen Schichten im dreidimensionalen Modus, welches das dreidimensionale Gitterprofil der anatomischen Sollform des Implantats wiedergibt, hier das Höhenprofil des Orbitabodens.

Weiter mit Bezug auf Fig. 2b wird dann das ermittelte Stützpunktgitter SG räumlich in eine vorgegebene Orientierung zu einer Referenzfläche R gebracht, welche einer vorgegebenen Referenzeinstellung der Stützpunkte der Vorformeinrichtung entspricht. Im vorliegenden Fall ist die Referenzfläche R eine rechteckige, ebene Fläche.

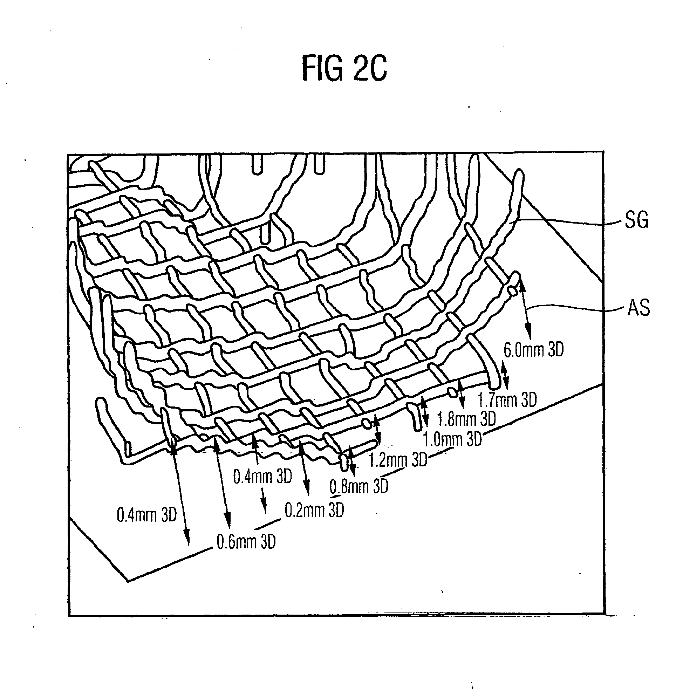

Weiter mit Bezug auf Fig. 2c erfolgt dann ein Ermitteln der Abstände zwischen der Referenzfläche R und dem Stützpunktgitter an den Stützpunkten in der Referenzfläche bei einer vorgegebenen Orientierung des Stützpunktgitters.

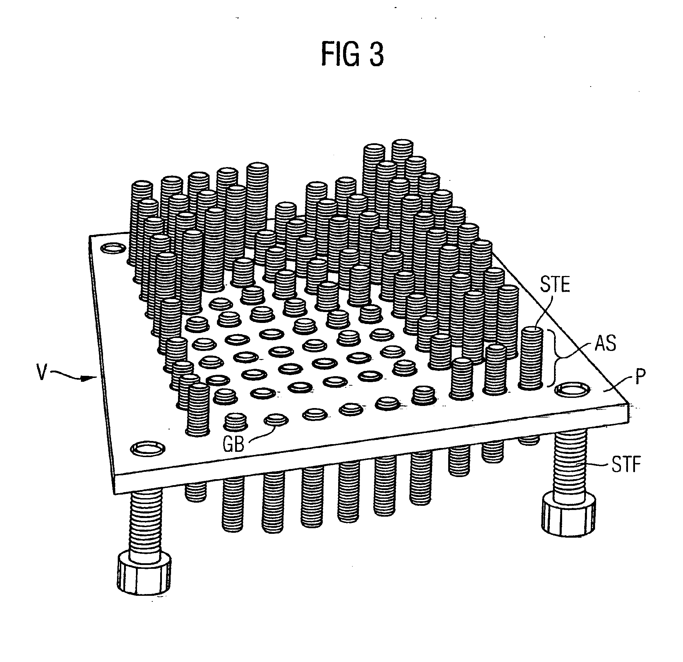

Dabei werden einfach Senkrechten von der Referenzfläche R zu den entsprechenden Punkten des Stützpunktgitters gezogen, also dort wo später die Stützeinrichtungen der nachstehend erläuterten Vorformeinrichtung aufliegen sollen. Die ermittelten Abstandswerte AS (oder deren relative Verhältnisse) sind dann identisch mit den Einstellungen der Stützeinrichtungen STE der Vorformeinrichtung V, die in Fig. 3 illustriert ist.

Bei dem vorliegenden Beispiel besteht die Vorformeinrichtung V aus einer rechteckigen Platteneinrichtung P, welche eine rechteckige Matrix von Gewindebohrungen GB aufweist, durch die höhenverstellbar Zylinderschrauben STE geführt sind. Die Platteneinrichtung P ruht dabei auf Stützfüssen STF, welche eine Bodenfreiheit für die eingeschraubten Zylinderschrauben STE gewährleisten.

Hat man sämtliche gemäss Fig. 2c ermittelten Abstandswerte AS in Schraubhöhen der Zylinderschrauben STE umgesetzt, so ist die Einstellung der Vorformeinrichtung V beendet, und ein Stützpunktmodell der anatomischen Sollstruktur des Implantats liegt vor.

Nach der Justierung der Vorformeinrichtung V erfolgt das Anbiegen des Implantats I, wobei zur besseren Gestaltung kann vor dem Anbiegen eine dünne verformbare Folieneinrichtung oder eine angeformte Manteleinrichtung auf die eingestellten Stützeinrichtungen in Form der Zylinderschrauben STE der Vorformeinrichtung V gebracht werden, beispielsweise eine Aluminiumfolie oder eine Gummifolie oder ein Kunststoffmantel. Dies bewirkt, dass die Übergänge zwischen den Stützpunkten besser angepasst sind und keine ungewünschten Ausbeulungen durch den Anpressdruck aufweisen.

Nach dem Anbiegen des Implantats I oder vor dem Anbiegen des Implantats I kann auch ein Zuschneiden des Randes des Implantats I vorgenommen werden, um eine exakte Anpassung in die zu operierende anatomische Struktur gewährleisten.

Das Ausrichten des Implantats I während der Operation erfolgt nämlich anhand der anatomischen Randbegrenzung, insbesondere beim vorliegenden Beispiel vorderer und seitlicher Orbitarand, welcher intraoperativ auch leicht wiederzufinden ist.

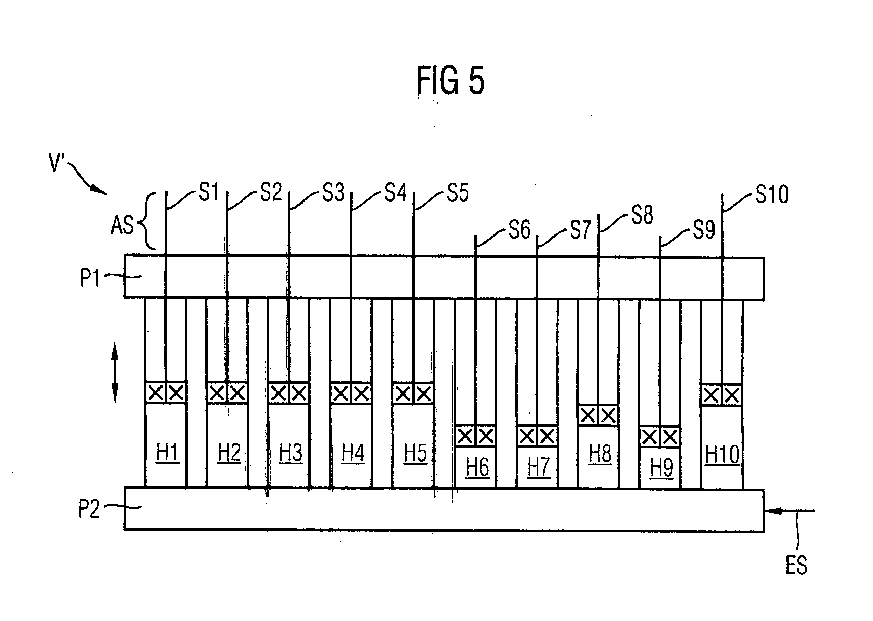

Fig. 5 zeigt eine weitere Ausführungsform der Vorformeinrichtung V', welche eine automatische Einstellung der Stützeinrichtungen S1-S10, hier dünne zylindrische Stifte ohne Gewinde, ermöglicht.

P1 bzw. P2 bezeichnen beim vorliegenden Beispiel eine obere Platteneinrichtung bzw. eine untere Platteneinrichtung. Zwischen der oberen Platteneinrichtung P1 und der unteren Platteneinrichtung P2 sind Höhenverstelleinrichtungen H1 - H10 vorgesehen, welche ansprechend auf elektronisches Einstellsignal ES die Abstände AS der Stützeinrichtungen S1 - S10 von der oberen Platteneinrichtung P1 einstellen. Die Höheneinstelleinrichtungen H1-H10 sind im vorliegenden Fall elektromagnetische Solenoide, können jedoch auch pneumatische oder sonstige mechanische Höheneinstelleinrichtungen sein.

Obwohl die vorliegende Erfindung anhand eines bevorzugten Ausführungsbeispiels vorstehend beschrieben wurde, ist sie darauf nicht beschränkt, sondern auf vielfältige Weise im Rahmendes Gegenstandes der Ansprüche modifizierbar.

Die vorliegende Erfindung ist nicht auf die besondere Gestalt der Vorformeinrichtung begrenzt. Insbesondere müssen die Stützeinrichtungen nicht unbedingt alle von einer ebenen Referenzfläche ausgehen, sondern auch von einer dreidimensionalen Referenzfläche ausgehen, wenn beispielsweise die Vorformeinrichtung für stark gekrümmte anatomische Sollformen verwendet wird.

Auch ist es nicht unbedingt notwendig, bei jeder Operation die anatomische Sollform exakt mittels computer-tomographischer oder ähnlicher Verfahren neu festzulegen, sondern nach Erhebung einer statistisch signifikanten Anzahl von Datensätzen für die Einstellungen können diese in einer Datenbank abgespeichert werden und standardisierte Schablonen der anatomischen Sollstruktur angelegt werden. Als Auswahlparameter können dabei beispielsweise Geschlecht, Alter, Rasse, Grösse, Körperseite, usw. vorgesehen werden.

Auch sei erwähnt, dass die Implantate aus den verschiedensten Materialien bestehen können, beispielsweise aus Titan oder aus Titan mit einem Überzug aus einem resorbierbaren weichen Material, z. B. PDS-Folie. Prinzipiell ist die vorliegende Erfindung jedenfalls nicht auf das spezielle Material des Implantats beschränkt, solange das Material in einem Anbiegeprozess verformbar ist.

Schliesslich sei erwähnt, dass das Einstellen der Stützeinrichtungen für die Vorformeinrichtung nicht mechanisch reversibel bzw. änderbar sein muss, sondern bei grossindustrieller Fertigung von grossen Stückzahlen desselben Standardimplantats auch irreversibel sein kann. In diesem Fall kann die Einstellung der Stützeinrichtungen z.B. durch Modellieren von Kunststoff o.ä. erfolgen. Dies gilt insbesondere dann, wenn vor dem Anbiegen eine permanent angeformte Manteleinrichtung auf die eingestellten Stützeinrichtungen der Vorformeinrichtung aufgebracht wird.

Auch kann der bereitgestellte Datensatz zum Gestalten der Vorformeinrichtung zur Herstellung eines entsprechend einer anatomischen Sollform vorgeformten flächigen Implantats für einen menschlichen oder tierischen Körper mit den Schritten dazu verwendet werden, eine kontinuierliche Formgebung z.B. durch Fräsen oder Giessen verwendet werden.

## Patentansprüche

1. Verfahren zur Herstellung eines entsprechend einer anatomischen Sollform vorgeformten flächigen Implantats (I) für einen menschlichen oder tierischen Körper mit den Schritten:
Bereitstellen eines Rohlings des flächigen Implantats;
Bereitstellen einer Vorformeinrichtung (V), welche eine Mehrzahl diskreter einstellbarer Stützeinrichtungen (STE) zum Einstellen einer zugehörigen Mehrzahl von Stützpunkten entsprechend der anatomischen Sollform aufweist;
Vorgeben der anatomischen Sollform und Ermitteln entsprechender Einstellungen für die Stützeinrichtungen (STE) ;
Einstellen der Stützeinrichtungen (STE) entsprechend der für die anatomische Sollform ermittelten Einstellungen; und
Bilden des vorgeformten Implantats (I) durch Anbiegen des Rohlings an die eingestellten Stützeinrichtungen (STE).

2. Verfahren nach Anspruch 1, **dadurch gekennzeichnet, dass** der Rohling eine zweidimensionale Gitterstruktur aufweist, welche durch das Anbiegen in eine dreidimensionale Gitterstruktur überführt wird.

3. Verfahren nach Anspruch 1 oder 2, **dadurch gekennzeichnet, dass** das Ermitteln der Einstellungen für die Stützeinrichtungen (STE; S1-S10) durch folgende Schritte erfolgt:
Abbilden eines der anatomischen Sollform entsprechenden Bereichs des Körpers in ein Stützpunktgitter (SG) ;
Ermitteln der Abstände zwischen einer Referenzfläche (R) entsprechend einer vorgegebenen Referenzeinstellung der Stützpunkte der Vorformeinrichtung (V; V') und dem Stützpunktgitter (SG) an den Stützpunkten in der Referenzfläche (R) bei einer vorgegebenen Orientierung des Stützpunktgitters (SG); und
Einstellen der ermittelten Abstände an den Stützeinrichtungen (STE; S1-S10).

4. Verfahren nach Anspruch 1, 2 oder 3, **dadurch gekennzeichnet, dass** das Abbilden des der anatomischen Sollform entsprechenden Bereichs des Körpers in ein Stützpunktgitter (SG) durch folgende Schritte erfolgt:
Abbilden eines der anatomischen Sollform gemäss einer mathematischen Abbildung entsprechenden Bereichs des Körpers in ein Abbild-Stützpunktgitter; und
Transformieren des Abbild-Stützpunktgitters in das Stützpunktgitter entsprechend der mathematischen Abbildung.

5. Verfahren nach Anspruch 3 oder 4, **dadurch gekennzeichnet, dass** das Abbilden mittels einer Computertomograhie-Vorrichtung durchgeführt wird.

6. Verfahren nach einem der vorhergehenden Ansprüche **dadurch gekennzeichnet, dass** das vorgeformte Implantat (I) eine vorgeformte Osteosynthese für die Orbita ist.

7. Verfahren nach einem der vorhergehenden Ansprüche **dadurch gekennzeichnet, dass** die Stützeinrichtungen (STE; S1-S10) zylindrische Stifteinrichtungen aufweisen, welche höhenverstellbar an einer ebenen Platteneinrichtung (P) angebracht sind.

8. Verfahren nach einem der vorhergehenden Ansprüche **dadurch gekennzeichnet, dass** das Einstellen der Stützeinrichtungen (STE; S1-S10) durch eine automatische Einstellungseinrichtung erfolgt.

9. Verfahren nach einem der vorhergehenden Ansprüche **dadurch gekennzeichnet, dass** vor dem Anbiegen eine dünne verformbare Folieneinrichtung oder eine angeformte Manteleinrichtung auf die eingestellten Stützeinrichtungen (STE; S1-S10) der Vorformeinrichtung (V, V') aufgebracht wird.

10. Verfahren nach einem der vorhergehenden Ansprüche **dadurch gekennzeichnet, dass** das Vorgeben der anatomischen Sollform und das Ermitteln der entsprechenden Einstellungen für die Stützeinrichtungen (STE; S1-S10) durch folgende Schritte erfolgt:
Vorgeben von Standard-Sollformen und entsprechenden Einstellungs-Datensätzen in einer Datenbank;
Auswählen eines Standard-Sollformen aus der Datenbank anhand eines oder mehrerer folgender Parameter: Geschlecht, Alter, Rasse, Grösse, Körperseite; und
Ermitteln der entsprechenden Einstellungen für die Stützeinrichtungen durch den zur ausgewählten Standard-Sollformen zugehörigen Einstellungs-Datensatz.

11. Verfahren nach einem der vorhergehenden Ansprüche **dadurch gekennzeichnet, dass** das Implantat (I) aus Titan besteht.

12. Vorrichtung zur Herstellung eines entsprechend einer anatomischen Sollform vorgeformten flächigen Implantats (I) für einen menschlichen oder tierischen Körper mit:
einer Vorformeinrichtung (V, V'), welche eine Mehrzahl diskreter einstellbarer Stützeinrichtungen (STE; S1-S10) zum Einstellen einer zugehörigen Mehrzahl von Stützpunkten entsprechend der anatomischen Sollform aufweist;
wobei die eingestellten Stützeinrichtungen (STE; S1-S10) ein Bilden des vorgeformten Implantats (I) durch Anbiegen eines Rohlings an die eingestellten Stützeinrichtungen (STE; S1-S10) ermöglichen.

13. Vorrichtung nach Anspruch 12 **dadurch gekennzeichnet, dass** die Stützeinrichtungen (STE) zylindrische Stifteinrichtungen aufweisen, welche verstellbar an einer ebenen Platteneinrichtung (P) angebracht sind.

14. Vorrichtung nach Anspruch 13 **dadurch gekennzeichnet, dass** die Stifteinrichtungen Schraubzylinder sind, die höhenverstellbar durch zugehörige Gewindebohrungen der Platteneinrichtung (P) geführt sind.

15. Vorrichtung nach einem der Ansprüche 12 bis 14, **gekennzeichnet durch** eine Einstellungseinrichtung zum automatischen Einstellen der Stützeinrichtungen (STE; S1-S10) anhand zugeführter Einstellungen.

16. Vorrichtung nach einem der Ansprüche 12 bis 15, **gekennzeichnet durch** eine Einstellungs-Ermittlungseinrichtung
zum Abbilden eines der anatomischen Sollform entsprechenden Bereichs des Körpers in ein Stützpunktgitter (SG); und
zum Ermitteln der Abstände zwischen einer Referenzfläche (R) entsprechend einer vorgegebenen Referenzeinstellung der Stützpunkte der Vorformeinrichtung (V; V') und dem Stützpunktgitter (SG)
an den Stützpunkten in der Referenzfläche (R) bei einer vorgegebenen Orientierung des Stützpunktgitters (SG) als die Einstellungen.

17. Vorrichtung nach Anspruch 16, **dadurch gekennzeichnet, dass** die Einstellungs-Ermittlungseinrichtung zum Abbilden des der anatomischen Sollform entsprechenden Bereichs des Körpers in ein Stützpunktgitter (SG) folgende Schritte automatisch ausführt:
Abbilden eines der anatomischen Sollform gemäss einer mathematischen Abbildung entsprechenden Bereichs des Körpers in ein Abbild-Stützpunktgitter; und
Transformieren des Abbild-Stützpunktgitters in das Stützpunktgitter entsprechend der mathematischen Abbildung.

18. Vorrichtung nach Anspruch 16 oder 17, **dadurch gekennzeichnet, dass** die Einstellungs-Ermittlungseinrichtung das Abbilden mittels einer Computertomograhie-Vorrichtung durchführt.

19. Vorrichtung nach einem der vorhergehenden Ansprüche **dadurch gekennzeichnet, dass** die Einstellungs-Ermittlungseinrichtung zum Abbilden des der anatomischen Sollform entsprechenden Bereichs des Körpers in ein Stützpunktgitter (SG) folgende Schritte automatisch ausführt:
Vorgeben von Standard-Sollformen und entsprechenden Einstellungs-Datensätzen in einer Datenbank;
Auswählen eines Standard-Sollformen aus der Datenbank anhand eines oder mehrerer folgender durch eine Eingabeeinrichtung empfangener Parameter: Geschlecht, Alter, Rasse, Grösse, Körperseite; und
Ermitteln der entsprechenden Einstellungen für die Stützeinrichtungen (STE; S1-S10) durch den zur ausgewählten Standard-Sollformen zugehörigen Einstellungs-Datensatz.

20. Verfahren zum Bereitstellen eines Datensatzes zum Gestalten einer Vorformeinrichtung (V, V') zur Herstellung eines entsprechend einer anatomischen Sollform vorgeformten flächigen Implantats (I) für einen menschlichen oder tierischen Körper mit den Schritten:
Abbilden eines der anatomischen Sollform entsprechenden Bereichs des Körpers in ein Stützpunktgitter (SG);
Ermitteln der Abstände zwischen einer vorgegebenen Referenzfläche (R) der Vorformeinrichtung (V; V') und dem Stützpunktgitter (SG) an den Stützpunkten in der Referenzfläche (R) bei einer vorgegebenen Orientierung des Stützpunktgitters (SG).

21. Verfahren nach Anspruch 20, **dadurch gekennzeichnet, dass** das Abbilden des der anatomischen Sollform entsprechenden Bereichs des Körpers in ein Stützpunktgitter (SG) durch folgende Schritte erfolgt:
Abbilden eines der anatomischen Sollform gemäss einer mathematischen Abbildung entsprechenden Bereichs des Körpers in ein Abbild-Stützpunktgitter; und
Transformieren des Abbild-Stützpunktgitters in das Stützpunktgitter entsprechend der mathematischen Abbildung.

22. Verfahren nach Anspruch 20 oder 21, **dadurch gekennzeichnet, dass** das Abbilden mittels einer Computertomograhie-Vorrichtung durchgeführt wird.

23. Verfahren nach Anspruch 20, **dadurch gekennzeichnet, dass** das Abbilden des der anatomischen Sollform entsprechenden Bereichs des Körpers in ein Stützpunktgitter (SG) durch folgende Schritte erfolgt:
Vorgeben von Standard-Sollformen und entsprechenden Einstellungs-Datensätzen in einer Datenbank;
Auswählen eines Standard-Sollformen aus der Datenbank anhand eines oder mehrerer folgender Parameter: Geschlecht, Alter, Rasse, Grösse, Körperseite; und
Festlegen des Stützpunktgitters (SG) durch den zur ausgewählten Standard-Sollformen zugehörigen Einstellungs-Datensatz.

24. Verfahren zum Gestalten einer Vorformeinrichtung zur Herstellung eines entsprechend einer anatomischen Sollform vorgeformten flächigen Implantats (I) für einen menschlichen oder tierischen Körper mit einem Datensatz gemäss einem der Ansprüche 20 bis 23, wobei aus dem Datensatz eine Oberflächentopologie für die Vorformeinrichtung (V; V') gebildet wird und dementsprechend die Vorformeinrichtung (V; V'), vorzugsweise durch Fräsen oder Giessen, hergestellt wird.

25. Verfahren nach Anspruch 24, **dadurch gekennzeichnet, dass** die Vorformeinrichtung (V; V') eine Mehrzahl diskreter einstellbarer Stützeinrichtungen (STE; S1-S10) zum Einstellen einer zugehörigen Mehrzahl von Stützpunkten entsprechend der anatomischen Sollform aufweist, wobei folgende Schritte ausgeführt werden:
Ermitteln entsprechender Einstellungen für die Stützeinrichtungen (STE; S1-S10) aus dem Datensatz; und
Einstellen der Stützeinrichtungen (STE; S1-S10) entsprechend der für die anatomische Sollform ermittelten Einstellungen.

26. Verfahren nach Anspruch 25, **dadurch gekennzeichnet, dass** eine dünne verformbare Folieneinrichtung oder eine angeformte Manteleinrichtung auf die eingestellten Stützeinrichtungen der Vorformeinrichtung (V; V') aufgebracht wird.

## Claims

1. A method for producing a sheet implant (I) for a human or animal body, which is preformed corresponding to a desired anatomical shape, with the steps of:
making available a blank of the sheet implant;
making available a preforming device (V), which has a plurality of discrete, adjustable supporting devices (STE) for adjusting an associated plurality of supporting points corresponding to the desired anatomical shape;
specifying the desired anatomical shape and determining corresponding settings for the supporting devices (STE);
setting the supporting devices (STE) to correspond to the settings determined for the desired anatomical shape; and
forming the preformed implant (I) by bending the blank against the set supporting devices (STE).

2. The method of claim 1, **characterized in that** the blank has a two-dimensional mesh structure, which is converted by bending into a three-dimensional mesh structure.

3. The method of claims 1 or 2, **characterized in that** the settings for the supporting devices (STE; S1-S10) are determined by the following steps:
imaging a region of the body corresponding to the desired anatomical shape in a supporting point mesh (SG);
determining the distances between a reference surface (R) corresponding to a specified reference setting of the supporting points of the preforming device (V;V') and the supporting mesh (SG) at the supporting points in the reference surface (R) for a specified orientation of the supporting point mesh (SG); and
setting the distances determined at the supporting devices (STE;S1-S10).

4. The method of claim 1, 2 or 3, **characterized in that** the imaging of the region of the body into a supporting point mesh (SG), which corresponds to the desired anatomical shape, takes place in the following steps:
imaging a region of the body corresponding to a mathematical image in an image supporting point mesh and
transforming the image supporting point mesh into the supporting point mesh corresponding to the mathematical image.

5. The method of claims 3 or 4, **characterized in that** the imaging is carried out by means of a computer tomography device.

6. The method of one of the preceding claims, **characterized in that** the preformed implant (I) is a preformed osteosynthesis for the orbit.

7. The method of one of the preceding claims, **characterized in that** the supporting devices (STE;S1-S10) have cylindrical pin devices, the height of which can be adjusted and which are mounted in a planar plate device (P).

8. The method of one of the preceding claims, **characterized in that** the setting of the supporting devices (STE;S1-S10) is accomplished by and automatic setting device.

9. The method of one of the preceding claims, **characterized in that**, before the bending procedure, a thin, deformable film device or an integrally molded casing device is mounted on the set supporting devices (STE;S1-S10) of the preforming device (V;V').

10. The method of one of the preceding claims, **characterized in that** the desired anatomical shape is specified and the corresponding settings for the supporting devices (STE;S1-S10) are determined by the following steps:
standard, desired shapes and corresponding adjustment data records are entered into a database;
standard, desirable shape is selected from the database by means of one or more of the following parameters: sex, age, race, height, side of body; and
the corresponding adjustments for the supporting devices are determined by means of the adjustment data record belonging to the desired standard shapes selected.

11. The method of one of the preceding claims, **characterized in that** the implant (I) consists of titanium.

12. A device for producing a sheet implant for a human or animal body, which is preformed corresponding to a desired anatomical shape, with:
a preforming device (V;V'), which has a plurality of discrete, adjustable supporting devices (STE;S1-S10) for adjusting an associated plurality of supporting points corresponding to the desired anatomical shape;
and the set supporting devices (STE;S1-S10) enable a preformed implant (I) to be formed by bending a blank against the set supporting devices (STE;S1-S10).

13. The device of claim 12, **characterized in that** the supporting devices (STE) have cylindrical pin devices, which are mounted adjustability at a planar plate device (P).

14. The device of claim 13, **characterized in that** the pin devices are screw-in cylinders, the height of which can be adjusted and which are guided by associated threaded boreholes of the plate device (P).

15. The device of one of the claims 12 to 14, **characterized by** a setting device for automatically setting the supporting devices (STE;S1-S10) by means of settings supplied.

16. The device of one of the claims 12 to 15, **characterized by** a setting-determining device
for imaging a region of the body, corresponding to a desired anatomical shape, in a supporting point mesh (SG) and
for determining the distances between a reference surface (R) corresponding to a specified reference setting of the supporting points of the preforming device (V;V') and the supporting point mesh (SG) at the supporting points in the reference surface for a specified orientation of the supporting point mesh as the settings.

17. The device of claim 16, **characterized in that** the setting-determining device for imaging the region of the body, corresponding to the desired anatomical shape, in a supporting point mesh (SG), automatically carries out the following steps:
imaging a region of the body, corresponding to the desired anatomical shape, in accordance with a mathematical image, in a supporting point mesh image and
transforming the supporting point mesh image into the supporting point mesh in accordance with the mathematical image.

18. The device of claim 16 or 17, **characterized in that** the setting-determining device carries out the imaging by means of a computer tomography device.

19. The device of one of the preceding claims, **characterized in that** the setting-determining device for imaging the region of the body, corresponding to the desired anatomical shape, in a supporting point mesh (SG), carries out the following steps automatically:
specifying desired standard shapes and corresponding adjustment data records in a database;
selecting a desired standard shape from the data base by means of one or more of the following parameters, received by an input device: sex, age, race, height, side of body; and
determining the corresponding adjustments for the supporting devices (STE;S1-S10) by means of the adjustment data record belonging to the desired standard shapes selected.

20. A method for making available a data record for configuring a preforming device (V;V') for producing a sheet implant (I), preformed in accordance with a desired anatomical shape, for a human or animal body with the steps of:
imaging a region of the body, corresponding to the desired anatomical shape, in a supporting point mesh (SG);
determining the distances between a specified reference surface (R) of the preforming device (V;V') and the supporting point mesh (SG) at the supporting points in the reference surface (R) for a given orientation of the supporting point mesh (SG).

21. The method of claim 20, **characterized in that** the imaging of the region of the body, corresponding to the desired anatomical shape, in a supporting point mesh (SG) is accomplished by the following steps:
imaging a region of the body, corresponding to the desired anatomical shape according to a mathematical image, in a supporting point mesh image and
transforming the supporting point mesh copy into the supporting point mesh in accordance with the mathematical image.

22. The method of claims 20 or 21, **characterized in that** the imaging is carried out by means of a computer tomography device.

23. The method of claim 20, **characterized in that** the imaging of the region of the body, corresponding to the desired anatomical shape, in a supporting point mesh (SG), is accomplished by the following steps:
specifying desired standard shapes and corresponding setting data records in a database;
selecting a desired standard shape from the database by means of one or more of the following parameters: sex, age, race, height, side of the body; and
establishing the supporting point mesh (SG) by the setting data record belonging to the selected desired standard shapes.

24. Method for configuring a preformed device for producing a sheet implant (I) for a human or animal body, corresponding to a desired anatomical shape, with a data record according to one of the claims 20 to 23, a surface topology for the preforming device (V;V') being formed from the data record and, correspondingly, the preforming device (V;V) being produced preferably by milling or casting.

25. The method of claim 24, **characterized in that** the preforming device (V;V') has a plurality of discrete adjustable supporting devices (STE;S1-S10) for adjusting an associated plurality of supporting points corresponding to the desired anatomical shape, the following steps being carried out:
determining corresponding settings for the supporting devices (STE;S1-S10) from the data record; and
setting the supporting devices (STE;S1-S10) in accordance with the settings determined for the desired anatomical shape.

26. The method of claim 25, **characterized in that** a thin, deformable film device or an integrally molded casing device is mounted on the set supporting devices of the preforming device (V;V').

## Revendications

1. Procédé de fabrication d'un implant (I) en nappe préformé conformément à une forme de consigne anatomique et destiné à un corps humain ou animal, comprenant les étapes suivantes :
préparation d'une ébauche de l'implant en nappe ;
préparation d'un équipement de préformage (V) qui présente une multiplicité d'équipements d'appui (STE) réglables discrets pour le réglage d'une multiplicité correspondante de points d'appui conformément à la forme de consigne anatomique ;
définition préalable de la forme de consigne anatomique et détermination de réglages correspondants pour les équipements d'appui (STE) ;
réglage des équipements d'appui (STE) conformément aux réglages déterminés pour la forme de consigne anatomique ; et
formation de l'implant (I) préformé par pliage de l'ébauche sur les équipements d'appui (STE) réglés.

2. Procédé selon la revendication 1, **caractérisé en ce que** l'ébauche présente une structure treillissée à deux dimensions qui est convertie par pliage en une structure treillissée à trois dimensions.

3. Procédé selon la revendication 1 ou 2, **caractérisé en ce que** la détermination des réglages pour les équipements d'appui (STE ; S1-S10) est effectuée par les étapes suivantes :
reproduction, dans un treillis de points d'appui (SG), d'une zone du corps correspondant à la forme de consigne anatomique ;
détermination des intervalles entre une surface de référence (R) conformément à un réglage de référence prédéfini des points d'appui de l'équipement de préformage (V ; V') et le treillis de points d'appui (SG) au niveau des points d'appui dans la surface de référence (R) dans le cas d'une orientation prédéfinie du treillis de points d'appui (SG) ; et
réglage des intervalles déterminés au niveau des équipements d'appui (STE ; S1-S10).

4. Procédé selon la revendication 1, 2 ou 3, **caractérisé en ce que** la reproduction, dans un treillis de points d'appui (SG), de la zone du corps correspondant à la forme de consigne anatomique est effectuée par les étapes suivantes :
reproduction, dans un treillis de points d'appui de reproduction, d'une zone du corps correspondant à la forme de consigne anatomique selon une reproduction mathématique ; et
transformation du treillis de points d'appui de reproduction en treillis de points d'appui conformément à la reproduction mathématique.

5. Procédé selon la revendication 3 ou 4, **caractérisé en ce que** la reproduction est effectuée au moyen d'un dispositif de tomodensitométrie.

6. Procédé selon l'une des revendications précédentes, **caractérisé en ce que** l'implant (I) préformé est une ostéosynthèse préformée pour les orbites.

7. Procédé selon l'une des revendications précédentes, **caractérisé en ce que** les équipements d'appui (STE ; S1-S10) présentent des équipements de broches cylindriques qui sont mis en place de façon réglable hauteur sur un équipement de plaque plan (P).

8. Procédé selon l'une des revendications précédentes, **caractérisé en ce que** le réglage des équipements d'appui (STE ; S1-S10) est effectué par un équipement de réglage automatique.

9. Procédé selon l'une des revendications précédentes, **caractérisé en ce que**, avant le pliage, un mince équipement de feuille déformable ou un équipement d'enveloppe formé est appliqué sur les équipements d'appui réglés (STE ; S1-S10) de l'équipement de préformage (V ; V').

10. Procédé selon l'une des revendications précédentes, **caractérisé en ce que** la définition préalable de la forme de consigne anatomique et la détermination des réglages correspondants pour les équipements d'appui (STE ; S1-S10) sont effectuées par les étapes suivantes :
définition préalable de formes de consigne standards et de jeux de données de réglage correspondants dans une base de données ;
sélection d'une forme de consigne standard à partir de la base de données à l'aide d'un ou de plusieurs des paramètres suivants : sexe, âge, race, taille, côté du corps ; et
détermination des réglages correspondants pour les équipements d'appui par le jeu de données de réglage appartenant aux formes de consigne standards sélectionnées.

11. Procédé selon l'une des revendications précédentes, **caractérisé en ce que** l'implant (I) est constitué de titane.

12. Procédé de fabrication d'un implant (I) en nappe préformé conformément à une forme de consigne anatomique et destiné à un corps humain ou animal, avec :
un équipement de préformage (V ; V') qui présente une multiplicité d'équipements d'appui (STE ; S1-S10) réglables discrets pour le réglage d'une multiplicité correspondante de points d'appui conformément à la forme de consigne anatomique ;
les équipements d'appui réglés (STE ; S1-S10) permettant une formation de l'implant (I) préformé par le pliage d'une ébauche au niveau des équipements d'appui réglés (STE ; S1-S10).

13. Dispositif selon la revendication 12, **caractérisé en ce que** les équipements d'appui (STE) présentent des équipements de broches cylindriques qui sont mis en place de façon réglable au niveau d'un équipement de plaque plan (P).

14. Dispositif selon la revendication 13, **caractérisé en ce que** les équipements de broches sont des cylindres filetés qui sont guidés de façon réglable en hauteur à travers des alésages filetés correspondants de l'équipement de plaque (P).

15. Dispositif selon l'une des revendications 12 à 14, **caractérisé par** un équipement de réglage pour le réglage automatique des équipements d'appui (STE ; S1-S10) à l'aide de réglages introduits.

16. Dispositif selon l'une des revendications 12 à 15, **caractérisé par** un équipement de détermination de réglage
pour la reproduction, dans un treillis de points d'appui (SG), d'une zone du corps correspondant à la forme de consigne anatomique ; et
pour la détermination des intervalles entre une surface de référence (R) conformément à un réglage de référence prédéfini des points d'appui de l'équipement de préformage (V ; V') et le treillis de points d'appui (SG) au niveau des points d'appui dans la surface de référence (R) dans le cas d'une orientation prédéfinie du treillis de points d'appui (SG) en tant que réglages.

17. Dispositif selon la revendication 16, **caractérisé en ce que** l'équipement de détermination de réglage effectue automatiquement les étapes suivantes pour la reproduction, dans un treillis de points d'appui (SG), de la zone du corps correspondant à la forme de consigne anatomique :
reproduction, dans un treillis de points d'appui de reproduction, d'une zone du corps correspondant à la forme de consigne anatomique selon une reproduction mathématique ; et
transformation du treillis de points d'appui de reproduction en treillis de points d'appui conformément à la reproduction mathématique.

18. Dispositif selon la revendication 16 ou 17, **caractérisé en ce que** l'équipement de détermination de réglage effectue la reproduction au moyen d'un dispositif de tomodensitométrie.

19. Dispositif selon l'une des revendications précédentes, **caractérisé en ce que** l'équipement de détermination de réglage pour la reproduction, dans un treillis de points d'appui (SG), de la zone du corps correspondant à la forme de consigne anatomique effectue automatiquement les étapes suivantes :
définition préalable de formes de consigne standards et de jeux de données de réglage correspondants dans une base de données ;
sélection d'une forme de consigne standard à partir de la base de données à l'aide d'un ou de plusieurs des paramètres suivants reçus par un équipement d'entrée: sexe, âge, race, taille, côté du corps ; et
détermination des réglages correspondants pour les équipements d'appui (STE ; S1-10) par le jeu de données de réglage appartenant aux formes de consigne standards sélectionnées.

20. Procédé de préparation d'une base de données pour la configuration d'un équipement de préformage (V ; V') destiné à la fabrication d'un implant (I) en nappe préformé conformément à une forme de consigne anatomique et destiné à un corps humain ou animal, avec les étapes suivantes :
reproduction, dans un treillis de points d'appui (SG), d'une zone du corps correspondant à la forme de consigne anatomique ;
détermination des intervalles entre une surface de référence (R) prédéfinie de l'équipement de préformage (V ; V') et le treillis de points d'appui (SG) au niveau des points d'appui dans la surface de référence (R) dans le cas d'une orientation prédéfinie du treillis de points d'appui (SG).

21. Procédé selon la revendication 20, **caractérisé en ce que** la reproduction, dans un treillis de points d'appui (SG), de la zone du corps conformément à la forme de consigne anatomique est effectuée par les étapes suivantes :
reproduction, dans un treillis de points d'appui de reproduction, d'une zone du corps correspondant à la forme de consigne anatomique selon une reproduction mathématique ; et
transformation du treillis de points d'appui de reproduction en treillis de points d'appui conformément à la reproduction mathématique.

22. Procédé selon la revendication 20 ou 21, **caractérisé en ce que** la reproduction est effectuée au moyen d'un dispositif de tomodensitométrie.

23. Procédé selon la revendication 20, **caractérisé en ce que** la reproduction, dans un treillis de points d'appui (SG), de la zone du corps correspondant à la forme de consigne anatomique est effectuée par les étapes suivantes :
définition préalable de formes de consigne standards et de jeux de données de réglage correspondants dans une base de données ;
sélection d'une forme de consigne standard à partir de la base de données à l'aide d'un ou de plusieurs des paramètres suivants : sexe, âge, race, taille, côté du corps ;
stipulation du treillis de points d'appui (SG) par le jeu de données de réglage correspondant aux formes de consigne standards sélectionnées.

24. Procédé de configuration d'un équipement de préformage pour la fabrication d'un implant (I) en nappe préformé conformément à une forme de consigne anatomique pour un corps humain ou animal avec un jeu de données selon une des revendications 20 à 23, une topologie de surface étant formé à partir de la base de données pour l'équipement de préformage (V ; V'), et l'équipement de préformage (V ; V') étant fabriqué en conséquence, de préférence par fraisage ou moulage.

25. Procédé selon la revendication 24, **caractérisé en ce que** l'équipement de préformage (V ; V') présente une multiplicité d'équipements d'appui (STE) réglables discrets pour le réglage d'une multiplicité correspondante de points d'appui conformément à la forme de consigne anatomique, les étapes suivantes étant effectuées :
détermination de réglages correspondants pour les équipements d'appui (STE ; S1-S10) à partir de la base de données ; et
réglage des équipements d'appui (STE ; S1-S10) conformément aux réglages déterminés pour la forme de consigne anatomique.

26. Procédé selon la revendication 25, **caractérisé en ce qu'**un mince équipement de feuille déformable ou un équipement d'enveloppe formé est appliqué sur les équipements d'appui réglés de l'équipement de préformage (V ; V').
